# EUROPEAN PATENT APPLICATION

(11) **EP 3 733 936 A1**
(43) Date of publication of application: **04.11.2020**
(21) Application number: 18897080.0
(22) Date of filing: 14.12.2018
(51) Int. Cl.: C40B 40/06, C40B 50/06, C12N 15/113

(54) **PIG WHOLE GENOME SGRNA LIBRARY, AND CONSTRUCTION METHOD THEREFOR AND APPLICATION THEREOF**

(30) Priority: 29.12.2017 CN 201711490244
(71) Applicant: Genewiz. Inc Suzhou, Suzhou, Jiangsu 215123 (CN)
(72) Inventor: XU, Pengyang, Suzhou, Jiangsu 215123 (CN); XU, Fengdan, Suzhou, Jiangsu 215123 (CN); DUAN, Guangyou, Suzhou, Jiangsu 215123 (CN); MIN, Wenbo, Suzhou, Jiangsu 215123 (CN); XIA, Bugao, Suzhou, Jiangsu 215123 (CN); GE, Yi, Suzhou, Jiangsu 215123 (CN)
(74) Representative: Mewburn Ellis LLP
(86) International application number: PCT/CN2018/121326
(87) International publication number: WO 2019/128743

(57) **Abstract**

A pig genome-wide sgRNA library, a method for constructing the same, and a use thereof. The library includes sgRNA sequences of 20438 genes in a pig (Sus scrofus) whole genome, of which 17410 genes are designed to obtain 6 sgRNAs and 2828 genes are designed to obtain 1-5 sgRNAs. The sgRNAs are acquired by the following process: acquiring candidate sgRNAs from a target sequence, performing an off-target analysis on and scoring the candidate sgRNAs, and finally filtering sgRNAs according to a scoring result. Details and process in modules are optimized by using multiple design criteria and screening principles and finally the pig genome-wide sgRNA library is constructed.

## Description

### CROSS-REFERENCES TO RELATED APPLICATIONS

This application claims priority to Chinese patent application No. CN201711490244.2 filed with the CNIPA on December 29, 2017 and entitled "Pig whole genome sgRNA (small guide Ribonucleic Acid) library as well as construction method and application thereof", disclosure of which is incorporated herein by reference in its entirety.

### TECHNICAL FIELD

The present application relates to the field of genetic engineering and, in particular, to a pig genome-wide sgRNA library, a method for constructing the same, and a use thereof.

### BACKGROUND

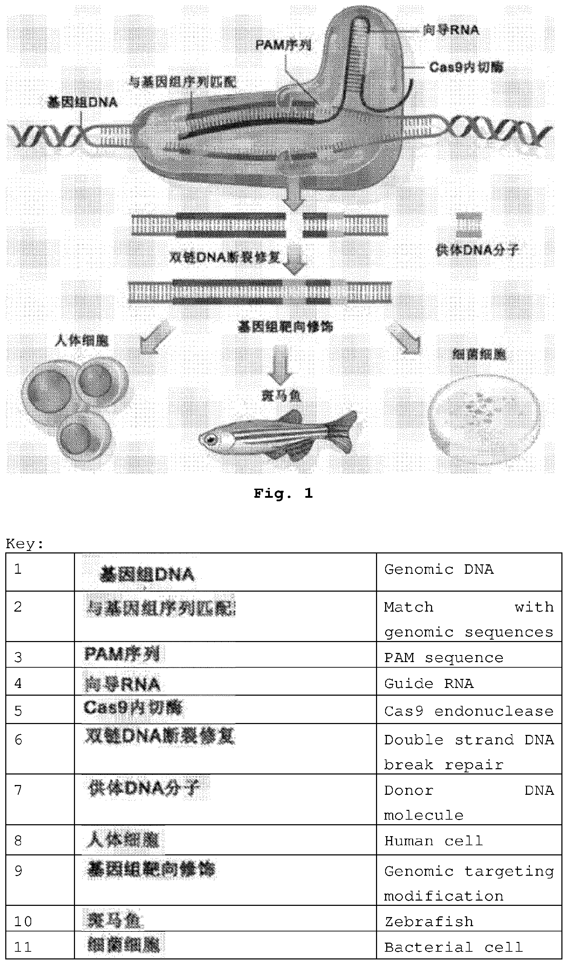

Clustered regularly interspaced short palindromic repeats (CRISPR) are in fact a kind of gene editor, a system of bacteria for protecting themselves against viruses, and also a genetic weapon against an attacker. Later, researchers discovered that the CRISPR seem to be an accurate universal genetic weapon that can be used for deleting, adding, activating or inhibiting target genes of other organisms, including genes in cells of humans, mice, zebrafish, bacteria, fruit flies, yeast, nematodes and crops, which also means that the gene editor is a widely applicable biotechnology. A working process of the CRISPR gene editor is shown in FIG 1.

A CRISPR cluster is a special family of repeated DNA sequences that are widely present in genomes of bacteria and archaea. Its sequence is composed of a leader, multiple short and highly-conserved repeats and multiple spacers. The leader is generally located upstream of the CRISPR cluster, a region rich in AT with a length of 300-500 bp, and regarded as a promoter sequence of the CRISPR cluster. The repeat has a length of 21-48 bp, contains a palindromic sequence, and can form a hairpin structure. The repeats are spaced by the spacer with a length of 26-72 bp. The spacer is composed of captured foreign DNA and similar to an immune memory. When foreign DNA containing a same sequence invades, the foreign DNA can be recognized by a bacterium body and cleaved to silence its expression, so as to protect the safety of the bacterium body.

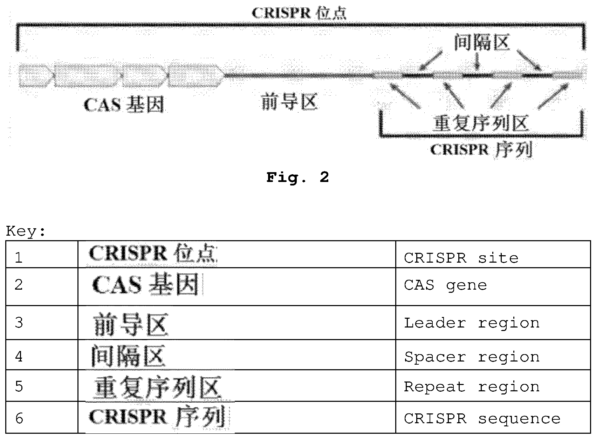

An analysis on flanking sequences of the CRISPR cluster revealed that a family of polymorphic genes exits nearby. Proteins encoded by the family all contain functional domains (with activity of nuclease, helicase, integrase and polymerase) that can interact with nucleic acids and work in collaboration with a CRISPR region. Therefore, the family of polymorphic genes is referred to as CRISPR associated (Cas) genes. Currently discovered Cas includes various types such as Cas1 to Cas10. The Cas genes and the CRISPR evolve together to form a highly-conserved system. A system structure of the CRISPR cluster is shown in FIG. 2.

When bacteria defend against the invasion of foreign DNA such as phages, under the control of the leader, the CRISPR is transcribed into a long RNA precursor (pre RISPR RNA (pre-crRNA)), then processed into a series of short mature crRNAs containing conserved repeated sequences and spacers, and recognized and bound to a complementary foreign DNA sequence to play a cleavage role.

It is currently discovered that a CRISPR/Cas system includes three different types, i.e., type I, type II and type III, which are present in approximately 40% of sequenced true bacteria and 90% of sequenced archaea. Type II has a relatively simple composition with Cas9 protein and a guide RNA (gRNA) as the core, and is most deeply studied at present.

In a type II system, the pre-crRNA is processed solely by Cas9 in the Cas family. Cas9 contains RuvC at an amino terminus and HNH2 unique active sites in the middle of protein and plays a role in crRNA maturation and double-stranded DNA cleavage. In addition, while the pre-crRNA is transcribed, a trans-activating crRNA (tracrRNA) complementary to repeated sequences of the pre-crRNA is also transcribed, and Cas9 and double-stranded RNA-specific nuclease RNase III are stimulated to process the pre-crRNA. After processing and maturation, the crRNA, the tracrRNA and Cas9 form a complex that recognizes and is bound to a complementary sequence of the crRNA, and DNA double strands are unwound to form an R-loop, such that the crRNA is hybridized with a complementary strand and the other strand remains in a free single-strand state. A complementary DNA strand of the crRNA is cleaved by HNH active sites in Cas9, a non-complementary strand is cleaved by an RuvC active site, and finally a DNA double-strand break (DSB) is introduced. A cleavage site of the CRISPR/Cas9 is located at an NGG site in a 5'-GG-N18-NGG-3' characteristic region of a protospacer adjacent motif (PAM) region adjacent to the downstream of the complementary sequence of the crRNA, and a sequence with this characteristic is repeated once in each random DNA sequence of 128bp. Research results showed that Cas9 can also cleave linear and supercoiled plasmids with cleavage efficiency comparable to a restriction enzyme. Since the crRNA participates and plays an accurate guiding role, the CRISPR/Cas9 targeting system is also referred to as an RNA guided targeting system.

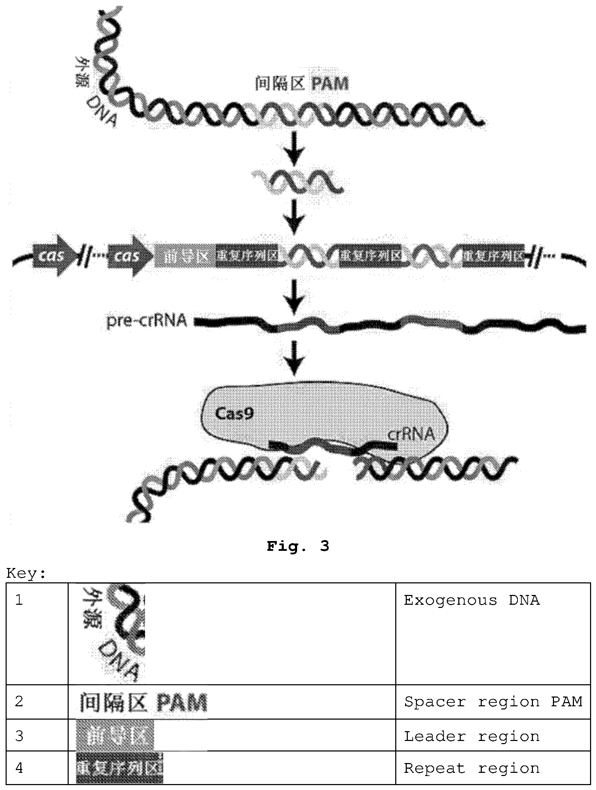

A principle of the targeting system is shown in FIG. 3.

A DNA editing system based on a CRISPR/Cas9-sgRNA has developed into an effective tool for editing genes. A CRISPR/Cas9-sgRNA system includes two main components: Cas9 protein and sgRNA. The sgRNA determines a gene editing site and gene editing efficiency. Researches have shown that different sgRNAs have different editing efficiency. Through high-throughput comparison and analysis of the efficiency of sgRNAs in animals and humans, researchers have obtained parameters of an efficient sgRNA.

CN106845151A has disclosed a method for screening sgRNA targets in a CRISPR-Cas9 system, which includes: (1) acquiring segments with a 5'-Nx-NGG-3' sequence in a genome (where x is an integer between 19 to 22 and N represents A/T/C/G) as candidate targets of an sgRNA in the CRISPR-Cas9 system by using genome-wide sequences and gene annotation information of published species; (2) breaking the genome into segments of 22-25 bp and screening sequences ending with NGG and having no repeats in the genome; (3) aligning sequences of the candidate targets in step (1) with the sequences screened in step (2), and screening and ordering preferred sequences according to mismatch information and a selection formula, to obtain a best set of genome-wide sgRNA targets. CN105886616A has disclosed an efficient and specific sgRNA recognition site guiding sequence for pig gene editing and a screening method thereof. The screening method includes the following steps: functional gene screening and an ORF analysis, functional gene sgRNA recognition site guiding sequence prediction, genome-wide off-target site detection, scoring predicted target sites based on off-target information and positions of target sites, ordering, result screening and statistics, and algorithm optimization and software development. A pig-specific sgRNA recognition site guiding sequence of the present application has undergone rigorous screening and testing and includes all sgRNA recognition site guiding sequences of pig protein-encoding genes for CRISPR-Cas9 gene editing. However, in the existing art, construction steps are cumbersome, optimization and screening criteria are backward, and the obtained sgRNAs are not high in quality.

However, low-quality sgRNAs directly waste researchers' time and money, and an effective way to avoid this result is to select efficient sgRNAs. At present, although there is some sgRNA design software, most of these software design sgRNAs gene by gene and lack a bioinformatics workflow of a design for customizing a genome-wide sgRNA library. Therefore, to provide a method for preparing an sgRNA library customized for a pig whole genome to obtain high-quality pig genome-wide sgRNAs has important scientific research values and application prospects.

### SUMMARY

In view of the deficiencies of the existing art and practical requirements, the present application provides a pig genome-wide sgRNA library, a method for constructing the same, and a use thereof. By adopting multiple design criteria and screening principles, algorithms and processes are optimized to finally construct a high-quality and highly active pig genome-wide sgRNA library. The method is concise and efficient, and the obtained library has a high quality and good activity, and is convenient for applications in gene editing researches.

To achieve the object, the present application adopts technical solutions described below.

In a first aspect, the present application provides a pig genome-wide sgRNA library, including sgRNA sequences of 20438 genes in a pig (Sus scrofus) whole genome.

The sgRNAs are mainly obtained by screening candidate sgRNAs on an acquired target sequence, performing an off-target analysis on and scoring the candidate sgRNAs, and finally filtering sgRNAs according to a scoring result.

Preferably, screening criteria for the candidate sgRNAs includes GC content of 20% to 80%.

The GC content is 20% to 80%, for example, may be 20%, 30%, 45%, 46%, 48%, 50%, 52%, 55%, 60%, 70% or 80%, preferably 40% to 70%.

Preferably, the screening criteria for the sgRNAs include a length of 19-21 nt.

The length is 19-21 nt, for example, may be 19 nt, 20 nt or 21 nt.

Preferably, filtering criteria for the sgRNAs include selecting an sgRNA close to a 5' end.

Preferably, the filtering criteria for the sgRNAs include that a number of sgRNAs for each CDS is not more than 2.

In a long-term process of conducting biological information sequencing experiments, the inventors have deeply researched a theoretical knowledge and a practical means of a CRISPR/Cas9-sgRNA DNA editing system, optimized an experimental process and algorithm modules and designed a large number of experiments to explore a design rule and a screening method of sgRNAs in order to construct a high-quality and highly-active sgRNA library that can meet experimental requirements, and finally found a best screening combination and filtering standard to obtain a specific sgRNA library customized for the pig whole genome.

In a second aspect, the present application provides a use of the pig genome-wide sgRNA library described in the first aspect for constructing a knockout-based mutant library and/or a knockout-based animal model.

In a third aspect, the present application provides a method for constructing a pig genome-wide sgRNA library. The method includes the following steps:
(1) selecting a target sequence: downloading genomic sequences and annotation files from a database, selecting a site with a 5'-20nt+NGG-3' sequence characteristic, and removing a sequence spanning an exon region as an input target sequence;
(2) designing sgRNAs: screening candidate sgRNAs according to GC content of 20% to 80% and a length of 19-21 nt, performing a genome-wide sequence alignment, and evaluating off-target rates and grading the sgRNAs according to a specified number of allowed mismatches; and
(3) filtering the sgRNAs: screening evaluated and graded sgRNAs according to the following criteria: selecting an sgRNA close to a 5' end and a number of sgRNAs for each CDS being not more than 2.

Preferably, a selection criterion of the target sequence in step (1) includes that a CDS region is selected as the target sequence for a protein-encoding gene.

Preferably, a basis for the screening in step (2) further includes a PAM sequence and a single/double-strand mode.

Preferably, the number of allowed mismatches in step (2) is 0 to 5, for example, may be 0, 1, 2, 3, 4 or 5, preferably 5.

Preferably, off-target rate evaluation criteria for designing the sgRNAs in step (2) include:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to a mismatch position of 5'->3', and comprehensively scoring the other sgRNAs in conjunction with a number of mismatches, where a larger penalty point corresponds to a higher risk.

Preferably, levels for the grading in step (2) include four levels: best, low-risk, moderate-risk and high-risk.

Preferably, the criteria for the screening in step (3) further include any one or a combination of at least two of: selecting 6 or less sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and the GC content of 20% to 80%, for example, may be a combination of selecting 6 or less sgRNAs for each target sequence and reserving only the best sgRNA and the low-risk sgRNA, a combination of the plurality of sgRNAs of each gene being targeted to the different positions of the each gene as much as possible and the GC content of 20% to 80%, a combination of selecting 6 or less sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%, preferably, the combination of selecting 6 or less sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%.

As a preferred technical solution, the present application provides a method for constructing a pig genome-wide sgRNA library. The method specifically includes the following steps:
(1) selecting a target sequence: downloading genomic sequences and annotation files from a database, selecting a site with a 5'-20nt+NGG-3' sequence characteristic, and removing a sequence spanning an exon region as an input target sequence;
   where a CDS region is selected as the target sequence for a protein-encoding gene;
(2) designing sgRNAs: screening candidate sgRNAs according to a PAM sequence, a single/double-strand mode, GC content of 20% to 80% and a length of 19-21 nt, performing a genome-wide sequence alignment and a sequence homology analysis, and evaluating off-target rates and grading the sgRNAs as best, low-risk, moderate-risk and high-risk according to a number of mismatches specified as 0 to 5;
   where off-target rate evaluation criteria include:
   (a) filtering out an sgRNA capable of being accurately aligned to a plurality of sites in a genome;
   (b) an sgRNA that is only aligned to a position corresponding to the sgRNA in the genome being Best; and
   (c) for other sgRNAs, gradually decreasing a penalty point according to a mismatch position of 5'->3', and comprehensively scoring the other sgRNAs in conjunction with the number of mismatches, where a larger penalty point corresponds to a higher risk;
(3) filtering the sgRNAs: screening evaluated and graded sgRNAs according to the following criteria: selecting an sgRNA close to a 5' end, a number of sgRNAs for each CDS being not more than 2, selecting 6 or less sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and the GC content of 20% to 80%.

Compared with the existing art, the present application has the following beneficial effects:
(1) The sgRNA library provided by the present application is of high quality. The 20438 genes in the whole genome are designed to obtain corresponding sgRNAs, of which 17410 genes are designed to obtain 6 sgRNAs and 2828 genes are designed to obtain 1-5 sgRNAs. All sgRNAs have activity meeting requirements of subsequent experiments.
(2) The method for constructing a pig genome-wide sgRNA library provided by the present application is concise, efficient, and convenient for operations and practical applications. By optimizing processes and details in modules and adjusting design criteria and screening principles of sgRNAs, sgRNAs are designed and filtered comprehensively and specifically for the pig whole genome, saving time and labor and facilitating promotion and applications.

### BRIEF DESCRIPTION OF DRAWINGS

FIG. 1 is a diagram of a working process of a CRISPR gene editor of the present application;
FIG. 2 is a diagram of a system structure of a CRISPR cluster of the present application;
FIG. 3 is a diagram of a principle of a CRISPR/Cas9 targeting system of the present application; and
FIG. 4 is a flowchart for sgRNA construction of the present application.

### DETAILED DESCRIPTION

To further elaborate on the technical means adopted and the effects achieved in the present application, the technical solutions of the present application are further described below with reference to the drawings and specific embodiments, but the present application is not limited to the scope of the embodiments.

### Example 1

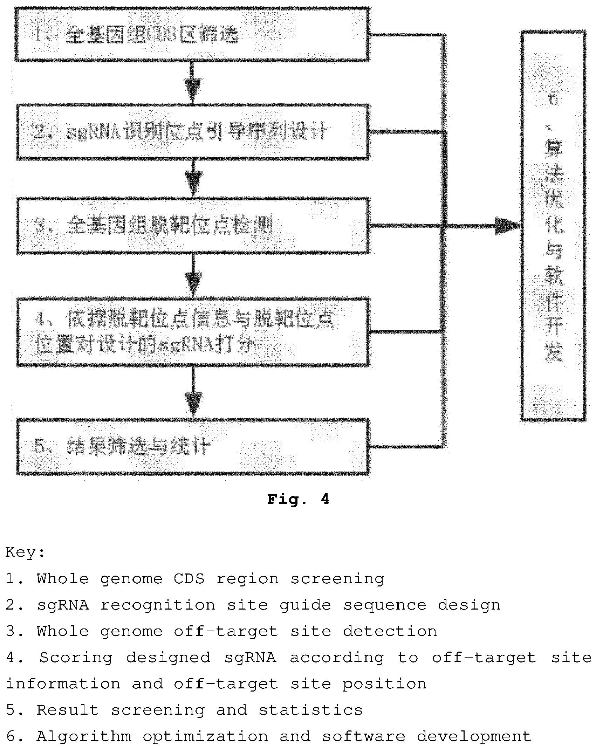

A construction process of sgRNAs is shown in FIG. 4. The process sequentially includes screening CDS sequences in the whole genome, selecting candidate sgRNA sequences according to sgRNA recognition sites, detecting off-target sites in the whole genome, scoring designed candidate sgRNAs according to information on the off-target sites and positions of the off-target sites, result screening and design, and algorithm optimization and software development in the whole process.

Specific steps are described below.
(1) A target sequence is selected: genomic sequences and annotation files are downloaded from a database, a site with a 5'20nt+NGG-3' sequence characteristic is selected, and a sequence spanning an exon region is removed as an input target sequence.
   A CDS region is selected as the target sequence for a protein-encoding gene.
(2) sgRNAs are designed: candidate sgRNAs are screened according to a PAM sequence, a single/double-strand mode, GC content of 20% to 80% and a length of 20 nt, a genome-wide sequence alignment is performed, and off-target rates are evaluated and the sgRNAs are graded as best, low-risk, moderate-risk and high-risk according to a number of mismatches specified as 5.
   Off-target rate evaluation criteria are described below.
   (a) An sgRNA capable of being accurately aligned to multiple sites in a genome is filtered out.
   (b) An sgRNA that is only aligned to a position corresponding to the sgRNA in the genome is Best.
   (c) For other sgRNAs, a penalty point is gradually decreased according to a mismatch position of 5'->3', and the other sgRNAs are comprehensively scored in conjunction with the number of mismatches, where a larger penalty point corresponds to a higher risk.
(3) sgRNAs are filtered: evaluated and graded sgRNAs are screened according to the following criteria: selecting an sgRNA close to a 5' end, a number of sgRNAs for each CDS being not more than 2, selecting 6 or less sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and the GC content of 20% to 80%.

In the constructed pig genome-wide sgRNA library, 20438 genes in total are designed to obtain sgRNAs, of which 17410 genes are designed to obtain 6 sgRNAs and 2828 genes are designed to obtain 1-5 sgRNAs. Results of experiments on sgRNA qualities show that the low-risk sgRNA and above are all high-quality sgRNAs, and sgRNAs in the constructed library all have activity that can meet requirements of subsequent experiments.

The applicant has stated that although the detailed method of the present application is described through the embodiments described above, the present application is not limited to the detailed method described above, which means that implementation of the present application does not necessarily depend on the detailed method described above. It should be apparent to those skilled in the art that any improvements made to the present application, equivalent replacements of various raw materials of the product, the addition of adjuvant ingredients, and the selection of specific manners, etc. in the present application all fall within the protection scope and the scope of disclosure of the present application.

## Claims

1. A pig genome-wide sgRNA library, comprising sgRNAs of 20438 genes in a pig (Sus scrofus) whole genome; wherein
the sgRNAs are acquired by the following process: acquiring candidate sgRNAs from a target sequence, performing an off-target analysis on and scoring the candidate sgRNAs, and finally filtering sgRNAs according to a scoring result.

2. The sgRNA library of claim 1, wherein screening criteria for the candidate sgRNAs comprise guanine-cytosine (GC) content of 20% to 80%;
preferably, the screening criteria for the candidate sgRNAs comprise GC content of 40% to 70%; and
preferably, the screening criteria for the candidate sgRNAs further comprise a length of 19-21 nt.

3. The sgRNA library of claim 1 or 2, wherein filtering criteria for the sgRNAs comprise selection of an sgRNA close to a 5' end;
preferably, the filtering criteria for the sgRNAs comprise that a number of sgRNAs for each commonly deleted segment (CDS) is not more than 2.

4. A use of the pig genome-wide sgRNA library of any one of claims 1 to 3 for constructing a knockout-based mutant library and/or a knockout-based animal model.

5. A method for constructing a pig genome-wide sgRNA library, comprising:
(1) selecting a target sequence: downloading genomic sequences and annotation files from a database, selecting a site with a 5'20nt+NGG-3' sequence characteristic, and removing a sequence spanning an exon region as an input target sequence;
(2) designing sgRNAs: screening candidate sgRNAs according to guanine-cytosine (GC) content of 20% to 80% and a length of 19-21 nt, performing a genome-wide sequence alignment, and evaluating off-target rates and grading the sgRNAs according to a specified number of allowed mismatches; and
(3) filtering the sgRNAs: screening evaluated and graded sgRNAs according to the following criteria: selecting an sgRNA close to a 5' end and a number of sgRNAs for each commonly deleted segment (CDS) being not more than 2.

6. The method of claim 5, wherein a selection criterion of the target sequence in step (1) comprises that a CDS region is selected as the target sequence for a protein-encoding gene;
preferably, a basis for the screening in step (2) further comprises a protospacer adjacent motif (PAM) sequence and a single/double-strand mode.

7. The method of claim 5 or 6, wherein the number of allowed mismatches in step (2) is 0 to 5, preferably 5;
preferably, off-target rate evaluation criteria for designing the sgRNAs in step (2) comprise:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to a mismatch position of 5'->3', and comprehensively scoring the other sgRNAs in conjunction with a number of mismatches, wherein a larger penalty point corresponds to a higher risk.

8. The method of any one of claims 4 to 7, wherein levels for the grading in step (2) comprise four levels: best, low-risk, moderate-risk and high-risk.

9. The method of any one of claims 5 to 8, wherein the criteria for the screening in step (3) further comprise any one or a combination of at least two of: selecting 6 or less sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and the GC content of 20% to 80%, preferably, a combination of selecting 6 or less sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%.

10. The method of any one of claims 5 to 9, specifically comprising:
(1) selecting a target sequence: downloading genomic sequences and annotation files from a database, selecting a site with a 5'-20nt+NGG-3' sequence characteristic, and removing a sequence spanning an exon region as an input target sequence;
wherein a CDS region is selected as the target sequence for a protein-encoding gene;
(2) designing sgRNAs: screening candidate sgRNAs according to a PAM sequence, a single/double-strand mode, GC content of 20% to 80% and a length of 19-21 nt, performing a genome-wide sequence alignment, and evaluating off-target rates and grading the sgRNAs as best, low-risk, moderate-risk and high-risk according to a number of mismatches specified as 0 to 5;
wherein off-target rate evaluation criteria comprise:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to a mismatch position of 5'->3', and comprehensively scoring the other sgRNAs in conjunction with the number of mismatches, wherein a larger penalty point corresponds to a higher risk;
(3) filtering the sgRNAs: screening evaluated and graded sgRNAs according to the following criteria: selecting an sgRNA close to a 5' end, a number of sgRNAs for each CDS being not more than 2, selecting 6 or less sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and the GC content of 20% to 80%.
